# FASCICULE DE BREVET EUROPEEN

(11) **EP 3 511 043 B1**
(45) Date de publication et mention de la délivrance du brevet: **19.08.2020**
(21) Numéro de dépôt: 18204428.9
(22) Date de dépôt: 05.11.2018
(51) Int. Cl.: A61M 16/00, A61B 5/08, A61H 31/00, A61B 5/083

(54) **APPAREIL DE VENTILATION POUR RÉANIMATION CARDIO-PULMONAIRE AVEC MONITORAGE ET AFFICHAGE DE LA VALEUR MAXIMALE DE CO2 MESURÉE**
BEATMUNGSGERÄT FÜR DIE HERZ-LUNGEN-WIEDERBELEBUNG MIT ÜBERWACHUNG UND ANZEIGE DES GEMESSENEN CO2-HÖCHSTWERTS
VENTILATION APPARATUS FOR CARDIOPULMONARY RESUSCITATION WITH MONITORING AND DISPLAY OF THE MEASURED MAXIMUM CO2 VALUE

(30) Priorité: 11.01.2018 FR 1850224
(43) Date de publication de la demande: 17.07.2019
(73) Titulaire: Air Liquide Medical Systems, 92160 Antony (FR)
(72) Inventeur: RIGOLLOT, Marceau, 92182 Antony (FR); RICHARD, Jean-Christophe, 92182 Antony (FR); BADAT, Bilal, 92182 Antony (FR)
(74) Mandataire: Pittis, Olivier

(56) Documents cités:
- WO-A1-2010/052608
- WO-A1-2012/114286
- WO-A1-2014/045182
- WO-A1-2014/072981
- WO-A1-2017/025869
- US-A1- 2016 256 102
- US-A1- 2016 287 170

## Description

L'invention porte sur un appareil d'assistance respiratoire, c'est-à-dire un ventilateur médical, pour fournir un gaz respiratoire à un patient recevant une réanimation cardio-pulmonaire (RCP), c'est-à-dire un patient en arrêt cardiaque soumis à un massage cardiaque avec alternance de compressions thoraciques et de relâchements, avec affichage de la valeur maximale de teneur en CO₂ mesurée pendant une période de temps donnée.

Les appareils médicaux de ventilation mécanique, encore appelés appareil d'assistance respiratoire ou ventilateurs médicaux, sont couramment utilisés pour fournir du gaz respiratoire, par exemple de l'air enrichi ou non en oxygène, à certains patients souffrant de troubles respiratoires.

La fourniture du gaz respiratoire au patient est couramment opérée au moyen d'une micro-soufflante motorisée et pilotée, comme notamment décrit par EP-A-3093498, EP-A-2947328, EP-A-2986856, EP-A-2954213 ou EP-A-2102504.

Il est connu de monitorer les composés gazeux présents dans le gaz administré aux patients, en particulier dans les gaz expirés par les patients qui contiennent du CO₂ résultant des échanges gazeux pulmonaires, c'est-à-dire du CO₂ produit par le métabolisme du patient, transporté jusqu'aux poumons grâce à la circulation sanguine puis évacué lors de l'expiration du patient. Ainsi, l'etCO₂ pour End Tidal CO₂ ou CO₂ en fin d'expiration, correspond à la mesure de la fraction de CO₂ expirée dans les gaz recueillis lors de l'expiration d'un individu, que son inspiration soit naturelle ou assistée c'est-à-dire obtenue par ventilation mécanique.

Sous ventilation mécanique, différentes techniques permettent l'analyse par spectrophotométrie de la fraction en CO₂ des gaz expirés. Pour ce faire, le gaz présent dans le circuit expiratoire peut être :
- soit aspiré puis analysé par une cellule d'analyse sur un site éloigné du circuit respiratoire. Cette manière d'opérer est appelée à flux dérivé ou secondaire, i.e. « *sidestream* » en anglais,
- soit analysé près du patient, préférentiellement au niveau d'une pièce Y agencée dans le circuit respiratoire à proximité du patient. Cette manière d'opérer est appelée à flux principal, i.e. « *mainstream* » en anglais.

Pendant une réanimation cardio-pulmonaire ou RCP pratiquée sur une personne en arrêt cardio-pulmonaire, avec mise en œuvre d'un massage cardiaque, le CO₂ alvéolaire, qui dépend non seulement des rapports ventilation/perfusion pulmonaire mais aussi de la quantité de CO₂ généré par le métabolisme cellulaire, représente un paramètre très utile au secouriste, par exemple un médecin, pour juger de l'efficacité de la RCP.

En théorie, plus la RCP est efficace, plus le débit cardiaque généré par les compressions thoraciques est important, plus la quantité de CO₂ ramenée vers les poumons est importante. Le monitorage de l'etCO₂, qui reflète indirectement le CO₂ alvéolaire, est de plus en plus utilisé pour monitorer la RCP de façon non invasive, c'est-à-dire pour informer le secouriste pendant qu'il opère le massage cardiaque, i.e., alternance de compressions thoraciques (CT) et de relâchements.

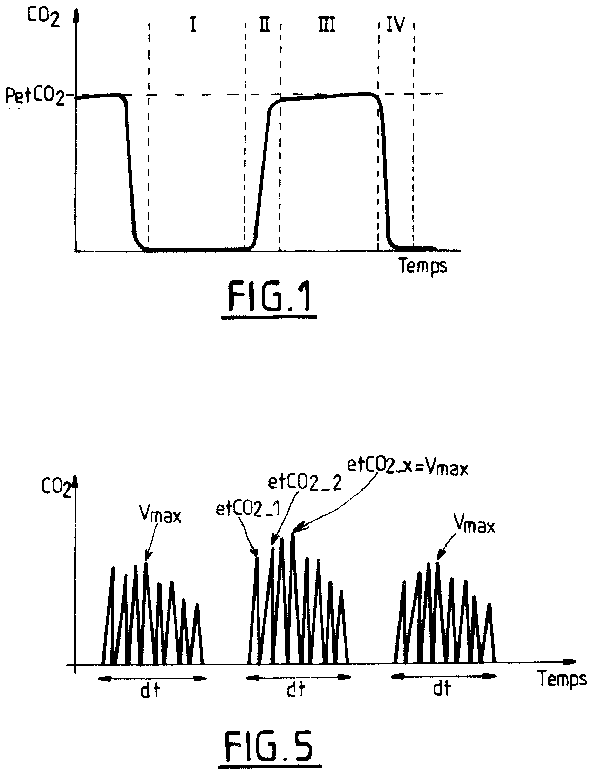

La Figure 1 est un capnogramme qui est une représentation graphique des variations de teneur en CO₂ dans les gaz respiratoires d'un patient au fil du temps (en secondes). Ce type de capnogramme est observé sur les patients ventilés en dehors des situations d'arrêt cardiaque. Comme on le voit, il se divise en quatre phases successives :
- Phase I : elle représente la ligne de base inspiratoire qui doit être stable à zéro.
- Phase II : elle est la partie ascendante du capnogramme et correspond à l'apparition du CO₂ dans les gaz expirés, au début de l'expiration du patient, par vidange des alvéoles les mieux ventilées. En réalité, l'expiration débute un peu avant cette phase car le gaz expiré en début d'expiration est dépourvu de CO₂ puisqu'il n'a pas participé aux échanges gazeux, du fait des espaces-morts instrumental et anatomique. L'augmentation en CO₂ est d'autant plus lente que le poumon est inhomogène et que les alvéoles ont des constantes de temps longues.
- Phase III : elle correspond à la phase de plateau alvéolaire qui correspond au gaz riche en CO₂ en provenance des alvéoles les moins bien ventilées. La valeur maximale de fin de plateau (PetCO2) correspond à la valeur d'etCO₂.
- Phase IV : elle correspond à la diminution de la concentration en CO₂ engendrée par un début d'inspiration spontanée ou assistée (i.e. mécanique).

Toutefois, lors d'une Réanimation Cardio-Pulmonaire (RCP) sur un patient en arrêt cardiorespiratoire, le capnogramme est très différent pour plusieurs raisons, notamment :
- Les compressions thoraciques génèrent des déplacements de petits volumes de gaz. Ces volumes, pourtant proches de l'espace mort instrumentale et anatomique, perturbent le capnogramme entre deux cycles ventilatoires. On observe ainsi souvent des tracés oscillants, la valeur max de CO₂ sur chaque compression thoracique (CT) ne cessant de changer.
- Les rapports ventilation/perfusion qui sont le reflet de la physiologie respiratoire sont très fortement modifiés. De plus, les petits tronçons de gaz mobilisés par les compressions thoraciques peuvent passer plusieurs fois devant le capteur. La concentration maximale observée, lors de chaque compression thoracique (CT), est donc souvent éloignées de la véritable concentration alvéolaire.
- Des comportements dynamiques d'ouverture et de fermeture des petites voies aériennes, lors de la RCP, ont été rapportés. Ce phénomène compromet les échanges gazeux et donc l'interprétation des concentrations de CO₂ lors de la RCP.

On comprend donc que l'etCO₂ tel qu'il est mesuré actuellement, c'est-à-dire lors de chaque compression thoracique (CT), ne permet pas d'obtenir une approximation fiable du CO₂ alvéolaire, alors que ce CO₂ alvéolaire est important car il peut être le reflet de la qualité de la RCP et d'une possible reprise d'activité cardiaque spontanée (RACS).

Le problème récurrent qui en résulte est qu'une mesure du CO₂ sans prise en compte de tout ou partie de ces facteurs, en particulier de l'impact de la ventilation réalisée sur le patient en arrêt cardiaque et de la variabilité du signal de CO2 entre deux cycles machine, rend l'utilisation de cette mesure de CO₂ peu fiable, voire inutilisable.

Les solutions actuelles de monitorage de l'etCO₂ sont adaptées aux variations de CO₂ provoquées par la respiration, qu'elle soit mécanique ou spontanée. Les fréquences en jeu sont de l'ordre de 10 à 40 c/min. Les algorithmes et mécanismes mis en œuvre sont adaptés à ces fréquences et à des variations faibles du CO₂ entre deux respirations du patient.

A ce titre, on peut citer les documents WO-A-14072981, US-A-2016/133160 et US-A-2012/016279, qui proposent des méthodes de suivi de la teneur en CO₂ dans les gaz expirés par un patient subissant une RCP, dans lesquelles les ventilateurs indiquent au secouriste qu'il doit stopper le massage cardiaque lorsque la teneur en etCO₂ est supérieure à 30 mm Hg par exemple.

Or, pendant une réanimation cardio-pulmonaire, les fréquences des compressions thoraciques (CTs) en jeu sont proches de 100 c/min, les volumes de gaz mobilisés faibles et les débits de gaz importants et irréguliers. De plus, le problème d'espace mort évoqué précédemment s'ajoute à ces difficultés puisque, du fait des CTs, une même fraction de gaz peut être analysée plusieurs fois par le capteur de CO₂, à défaut de mise en place d'un rinçage ou purge de l'espace mort.

Dans ces conditions, la valeur de l'etCO₂ affichée par les ventilateurs actuels est rafraîchie à une fréquence inadéquate puisque ceux-ci tentent de suivre l'évolution du CO₂ à la fréquence du massage, à savoir 100 c/min. En d'autres termes, les valeurs d'etCO₂ affichées par les ventilateurs actuels ne sont pas représentatives d'une concentration de CO₂ liée au métabolisme du patient car l'origine du gaz analysé n'est pas garantie. En d'autres termes, les valeurs mesurées sont souvent erronées car elles ne reflètent pas ou alors que très mal, la concentration en CO₂ alvéolaire.

On connait par ailleurs WO-A-2012/114286 qui enseigne un système de capnographie avec suivi du CO₂ servant à réaliser des diagnostics automatiques de l'effet d'une thérapie sur un patient, comme par exemple le bon positionnement d'un tube endotrachéal, l'efficacité d'une RCP ou un retour à une circulation spontanée (ROCS). Pour ce faire, le système discrimine les respirations réelles des artefacts, puis analyse la forme des ondes respiratoires, en particulier la ligne basale, l'amplitude, la fréquence, les pentes...

WO-A-2010/052608 décrit un système similaire permettant un suivi du CO₂ dans les gaz respiratoires durant une intubation qui est utilisable pendant une RCP ou une ventilation. Là encore, il est opéré une analyse de la forme des ondes respiratoires, en particulier la ligne basale, l'amplitude, la fréquence, les pentes..., et une alarme est déclenchée en cas de détection d'une onde anormale.

Le problème qui se pose dès lors est de proposer un appareil d'assistance respiratoire amélioré, c'est-à-dire un ventilateur médical, qui permette d'afficher, pendant une RCP mettant en œuvre l'appareil d'assistance respiratoire, une valeur de CO₂ fiable, c'est-à-dire qui reflète le plus possible le CO₂ alvéolaire, dans le but de mieux assister le secouriste durant la RCP en lui proposant une information pertinente facilitant le monitorage de la RCP, telle une détéction d'un RACS par exemple.

La solution de l'invention concerne alors un appareil d'assistance respiratoire, c'est-à-dire un ventilateur médical, pour fournir un gaz respiratoire, tel de l'oxygène, à un patient pendant une réanimation cardio-pulmonaire (RCP) comprenant :
- une source de gaz respiratoire pour fournir un gaz respiratoire au dit patient pendant une réanimation cardio-pulmonaire (RCP),
- des moyens de mesure de teneur en CO₂ pour opérer des mesures de concentration en CO₂ produit par le patient, et fournir des signaux de mesure de teneur en CO₂ à des moyens de traitement de signal et de pilotage,
- des moyens de traitement de signal et de pilotage configurés pour traiter les signaux de mesure de teneur en CO₂ provenant des moyens de mesure de teneur en CO₂,
- au moins une interface graphique utilisateur (IGU),
caractérisé en ce que :
- les moyens de traitement de signal et de pilotage sont configurés pour :
   a) traiter les signaux de mesure de teneur en CO₂ correspondant à des mesures opérées par les moyens de mesure de teneur en CO₂ pendant une période de temps (dt) donnée qui correspond à plusieurs contractions thoraciques et relâchements de compression successifs opérés par un secouriste réalisant la RCP sur le patient P en état d'arrêt cardiaque, et en extraire une pluralité de valeurs de pics de teneur en CO₂,
   b) sélectionner la valeur maximale (Vmax) de teneur en CO₂ parmi la pluralité de valeurs de pics de teneur en CO₂ mesurées pendant ladite période de temps (dt) donnée, et
   c) transmettre ladite valeur maximale (Vmax) de teneur en CO₂ à l'interface graphique utilisateur,
- et l'interface graphique utilisateur est configurée pour afficher la valeur maximale (Vmax) de teneur en CO₂.

Selon le cas, l'appareil d'assistance respiratoire de l'invention peut comprendre l'une ou plusieurs des caractéristiques techniques suivantes :
- l'IGU est configurée pour afficher au moins une valeur de teneur en CO₂ fournie par les moyens de traitement de signal et de pilotage.
- le CO₂ produit par le patient. Ce CO₂ est observable lors de l'expiration du patient et/ou être réinhalé à l'inspiration suivante, notamment lorsqu'il s'agit de gaz piégé entre une pièce Y et le capteur de CO₂ par exemple.
- la source de gaz respiratoire est en communication fluidique avec un conduit de gaz.
- la source de gaz respiratoire est une source d'air, en particulier une micro-soufflante motorisée, encore appelée turbine ou compresseur.
- les moyens de mesure de teneur en CO₂ sont agencés de manière à opérer des mesures de concentration en CO₂ en aval du conduit de gaz.
- les moyens de mesure de teneur en CO₂ sont reliés électriquement aux moyens de traitement de signal et de pilotage.
- la source de gaz respiratoire est en communication fluidique avec un conduit de gaz servant à véhiculer le gaz respiratoire vers le patient, i.e. jusqu'à une interface respiratoire.
- les moyens de traitement de signal et de pilotage comprennent au moins une carte électronique ou analogue.
- les moyens de traitement de signal et de pilotage comprennent au moins un microprocesseur, de préférence un microcontrôleur.
- le microprocesseur met en œuvre au moins un algorithme.
- les moyens de mesure de teneur en CO₂ sont agencés sur le flux principal de gaz, c'est-à-dire qu'ils sont en agencement de type *'mainstream'* (i.e. flux principal).
- les moyens de mesure de teneur en CO₂ comprennent un capnomètre.
- les moyens de mesure de teneur en CO₂ sont agencés de manière à opérer des mesures de concentration en CO₂ en aval du conduit de gaz, de préférence au niveau d'une extrémité aval du conduit de gaz,
- le conduit de gaz est en communication fluidique avec une interface respiratoire, encore appelée interface patient.
- l'interface respiratoire est une sonde d'intubation endotrachéale, un masque facial ou un masque laryngé, aussi appelé dispositif supra glottique, ou tout dispositif adéquat d'administration de gaz.
- l'interface respiratoire est de préférence une sonde d'intubation endotrachéale, couramment appelée 'sonde trachéale'.
- les moyens de mesure de teneur en CO₂ sont en amont et à proximité immédiate de l'interface respiratoire, c'est-à-dire proche de la bouche du patient.
- selon un premier mode de réalisation, les moyens de mesure de teneur en CO₂ sont agencés sur une pièce de jonction agencée en amont de l'interface respiratoire, de préférence entre l'interface respiratoire et l'extrémité aval du conduit de gaz, en particulier entre l'interface respiratoire et une pièce en Y comprenant des passages internes de gaz.
- de préférence, les moyens de mesure de teneur en CO₂ sont agencés sur une pièce de jonction comprenant un passage interne de gaz.
- selon un deuxième mode de réalisation, les moyens de mesure de teneur en CO₂ sont agencés dans l'appareil, c'est-à-dire dans la carcasse de l'appareil, en étant reliés, via un conduit de prélèvement de gaz ou analogue, à un site de prélèvement de gaz situé en amont et à proximité immédiate de l'interface respiratoire.
- en particulier, les moyens de mesure de teneur en CO₂ sont reliés fluidiquement à un site de prélèvement de gaz porté par une pièce de jonction, en particulier agencée entre l'interface respiratoire et le conduit de gaz, typiquement entre l'interface respiratoire et une extrémité aval dudit conduit de gaz.
- la pièce de jonction est raccordée fluidiquement entre la pièce de raccordement intermédiaire, c'est-à-dire une pièce en Y, et l'interface respiratoire, typiquement une sonde trachéale ou un masque.
- la branche inspiratoire, la branche expiratoire et l'interface respiratoire sont en communication fluidique entre elles.
- il comprend un circuit patient comprenant une branche inspiratoire permettant d'acheminer du gaz vers le patient.
- le circuit patient comprend en outre une branche expiratoire permettant d'évacuer le gaz expiré par le patient.
- la branche inspiratoire, la branche expiratoire et l'interface patient sont connectées fluidiquement et/ou mécaniquement, directement ou indirectement, à la pièce de raccordement intermédiaire, en particulier une pièce en Y.
- les moyens de mesure de teneur en CO₂ sont agencés de manière à opérer des mesures de concentration en CO₂ à l'entrée de la branche expiratoire ou à la sortie de la branche inspiratoire du circuit de gaz.
- la branche expiratoire communique fluidiquement avec l'atmosphère pour évacuer tout ou partie du gaz expiré par le patient, en particulier le gaz riche en CO₂.
- la branche inspiratoire et/ou la branche expiratoire comprennent des tuyaux flexibles.
- de préférence, tout ou partie du conduit de gaz formant tout ou partie de la branche inspiratoire du circuit de gaz est un tuyau flexible.
- les moyens de traitement de signal et de pilotage sont configurés pour commander la source de gaz respiratoire et délivrer le gaz respiratoire selon des cycles ventilatoires successifs.
- chaque cycle ventilatoire comprend une phase BP (D_{BP}) pendant laquelle le gaz est délivré par la micro-soufflante à une pression dite basse ou basse pression (BP), et une phase HP (D_{HP}) pendant laquelle le gaz est délivré par la micro-soufflante à une pression dite haute ou haute pression (HP), avec HP > BP.
- la micro-soufflante est pilotée pour fournir du gaz à une pression basse (BP) comprise entre 0 et 20 cm d'eau, de préférence entre 0 et 15 cm d'eau, préférentiellement encore 0 et 10 cm d'eau.
- la micro-soufflante est pilotée pour fournir du gaz à une pression haute (HP) comprise entre 5 et 60 cm d'eau, de préférence entre 5 et 45 cm d'eau, préférentiellement encore 5 et 30 cm d'eau (avec HP > BP).
- la phase BP a une durée supérieure à la phase HP.
- la phase BP a une durée comprise entre 2 et 10 secondes, typiquement de l'ordre de 3 à 6 secondes.
- la phase HP a une durée comprise entre 0,5 et 3 secondes, typiquement de l'ordre de 1 à 2 secondes.
- la période de temps donnée (dt) est de plusieurs secondes.
- la période de temps (dt) est comprise entre 2 et 10 secondes, typiquement de l'ordre de 3 à 6 secondes.
- la période de temps (dt) correspond à la durée de la phase BP de chaque cycle ventilatoire.
- la durée totale d'un cycle ventilatoire est comprise entre 3 et 10 secondes.
- la période de temps (dt) donnée englobe plusieurs durées de compressions thoraciques et de relâchements successifs, typiquement entre 5 et 12 compressions thoraciques.
- les moyens de mesure de teneur en CO₂ sont configurés pour opérer des mesures en continu.
- les moyens de mesure de teneur en CO₂ comprennent un capteur de CO₂.
- les moyens de mesure de teneur en CO₂ comprennent un capteur de CO₂ dont la prise de mesure est en communication fluidique avec l'intérieur ou lumen de la pièce de jonction agencée en amont de l'interface respiratoire.
- il comprend des moyens de mémorisation coopérant avec les moyens de traitement de signal et de pilotage pour mémoriser la pluralité de valeurs de teneur en CO₂ mesurées pendant la période de temps donnée.
- les moyens de mémorisation sont en outre configurés pour mémoriser les valeurs maximales (Vmax) de teneur en CO₂ successives, c'est-à-dire mesurées pendant des périodes de temps (dt) données successives.
- les moyens de mémorisation comprennent une mémoire flash ou de type disque dur.
- il comprend en outre des moyens de mesure de débit gazeux configurés pour opérer au moins une mesure, de préférence en continu, du débit du gaz expiré et/ou du débit de gaz inspiré et ou expiré par le patient. Le débit permet le monitorage et la surveillance des compressions thoraciques, ainsi que le calcul, le monitorage et la surveillance des volumes de gaz délivrés et expirés (ventilateur et CTs).
- les moyens de mesure de débit gazeux comprennent un capteur de débit.
- l'interface graphique utilisateur (IGU) comprend un écran digital, de préférence un écran tactile.
- l'interface graphique utilisateur est configurée pour afficher la valeur maximale (Vmax) de teneur en CO₂ sous forme d'une valeur numérique ou d'une représentation graphique s'affichant sur l'IGU, ou les deux.
- l'interface graphique utilisateur est configurée pour afficher plusieurs valeurs maximales (Vmax) de teneur en CO₂ successives sous forme d'une représentation graphique.
- l'interface graphique utilisateur est configurée pour afficher une ou plusieurs valeurs maximales (Vmax) de teneur en CO₂ sous forme d'une représentation graphique de type courbe, barre-graphe ou autre.
- l'écran comprend plusieurs touches activant différentes fonctions et/ou plusieurs zones ou fenêtres d'affichage.
- l'écran est du type à affichage en couleurs.
- alternativement, l'écran est du type à affichage en noir et blanc ou alors permet un passage d'un affichage en couleurs à un affichage en noir et blanc de manière à opérer des économies d'énergie.
- il comprend une source de courant électrique, par exemple une batterie ou analogue, de préférence une batterie rechargeable.
- il comprend des moyens d'alarme configurés pour se déclencher lorsque la valeur maximale de teneur en CO₂ excède une valeur-seuil.
- les moyens d'alarme comprennent une alarme sonore ou visuelle, ou les deux.
- les moyens d'alarme sont programmés pour se déclencher lorsque la valeur maximale (Vmax) de CO₂ mesurée, à un instant t, est telle que : [VmaxCO₂] > n x [MoyCO₂], où:
   - n est compris entre 1,25 et 4, de préférence entre 1,5 et 3, par exemple de l'ordre de 2
   - [VmaxCO₂] est la valeur maximale de teneur en CO₂ mesurée pendant une durée dt donnée, par exemple sur une durée dt comprise entre 2 et 10 secondes,
   - [MOyCO₂] est la valeur moyenne des valeurs maximales de teneur en CO₂ [VmaxCO_{2]} déterminées pour plusieurs durées dt successives comprises dans une fenêtre de temps (FT) donnée (FT> x.dt avec x ≥ 2), par exemple une période de 30 sec à 5 minutes, ou plus.
- il comprend une carcasse rigide comprenant la source de gaz respiratoire, les moyens de traitement de signal et de pilotage, la source de courant électrique et les moyens de mémorisation.
- la carcasse rigide est formée en tout ou en partie de polymère.
- l'interface graphique est agencée dans l'une des parois formant la carcasse du ventilateur.
- les moyens de mesure de teneur en CO₂ sont configurés pour opérer des mesures de concentration en CO₂ successives sur des périodes de temps (dt) successives, c'est-à-dire des périodes de temps (dt) espacées les unes des autres.
- les moyens de mesure de teneur en CO₂ sont configurés pour opérer des mesures de concentration en CO₂ successives sur des périodes de temps (dt) successives pendant des cycles ventilatoires successifs, en particulier pendant les phases BP de cycles ventilatoires successifs.

La présente description décrit aussi un procédé (i.e., une méthode) de suivi d'une réanimation cardio-pulmonaire (RCP) opéré sur un patient en arrêt cardiaque, dans lequel :
- on met en œuvre un appareil d'assistance respiratoire comprenant une source (1) de gaz respiratoire, telle une micro-soufflante, pour fournir un gaz respiratoire à un patient pendant une réanimation cardio-pulmonaire (RCP),
- on opère des mesures de concentration en CO₂ produit par ledit patient, par exemple au moyen d'un capnomètre,
- on traite les signaux de mesure de teneur en CO₂, par exemple au moyen de moyens de traitement de signal et de pilotage (5), telle un microprocesseur,
- on détermine une pluralité de valeurs de pics de teneur en CO₂ mesurées pendant une période de temps (dt) donnée qui correspond à plusieurs contractions thoraciques et relâchements de compression successifs opérés par un secouriste réalisant la RCP sur le patient en état d'arrêt cardiaque,
- on sélectionne la valeur maximale (Vmax) de teneur en CO₂ parmi la pluralité de valeurs de pics de teneur en CO2 mesurées pendant ladite période de temps (dt) donnée, et
- on affiche la valeur maximale (Vmax) de teneur en CO₂, pendant la période de temps (dt) donnée, sur une IGU.

L'invention va maintenant être mieux comprise grâce à la description détaillée suivante, faite à titre illustratif mais non limitatif, en référence aux figures annexées parmi lesquelles :
- la Figure 1 est une représentation graphique des variations de teneur en CO₂ dans les gaz respiratoires d'un patient ventilé hors situation d'arrêt cardiaque,
- la Figure 2 schématise un cycle ventilatoire à deux niveaux de pression pouvant être mis en œuvre par l'appareil de la Figure 6 pour ventiler un patient en arrêt cardio-pulmonaire au cours d'une RCP,
- la Figure 3 illustre les variations de pression observées par la machine à l'extrémité du circuit respiratoire chez un patient en arrêt cardio-pulmonaire au cours d'une RCP,
- la Figure 4 schématise la quantité de CO₂ mesurée par le capnomètre de l'appareil de la Figure 6 avant et après retour à une activité cardiaque spontanée,
- la Figure 5 schématise les pics de teneur en CO₂ pendant les cycles ventilatoires mis en œuvre pendant une RCP, et
- la Figure 6 est un schéma d'un mode de réalisation d'un appareil d'assistance respiratoire pour RCP selon l'invention,

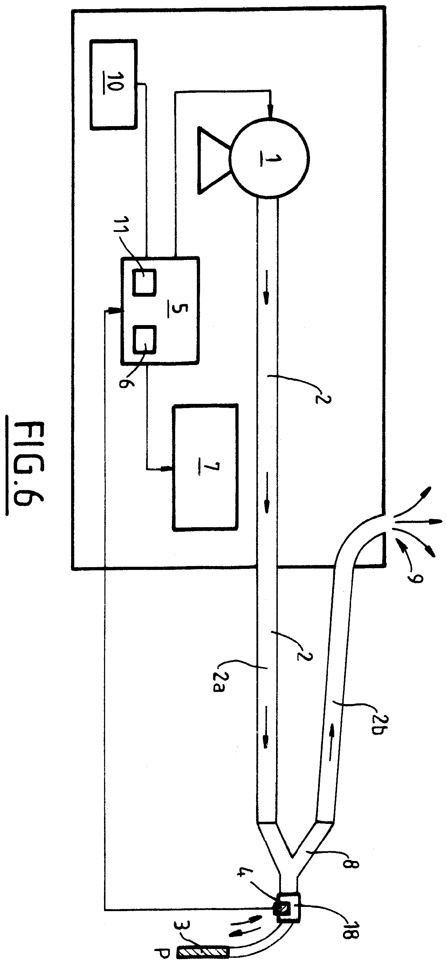

La Figure 6 est une représentation schématique d'un mode de réalisation d'un appareil d'assistance respiratoire ou ventilateur médical selon l'invention servant à fournir un gaz respiratoire, typiquement de l'air ou de l'air enrichi en oxygène, à un patient P pendant une réanimation cardio-pulmonaire (RCP), c'est-à-dire à une personne en arrêt cardiaque sur laquelle un secouriste pratique un massage cardiaque, c'est-à-dire une alternance de compressions thoraciques (CT) et de relâchements (Re), c'est-à-dire de non-compressions thoraciques.

Cet appareil ou ventilateur comprend une source 1 de gaz respiratoire, telle une micro-soufflante motorisée, qui est en communication fluidique avec un conduit de gaz 2 pour fournir un gaz respiratoire à audit patient P pendant la réanimation cardio-pulmonaire, typiquement de l'air sous pression.

La source 1 de gaz respiratoire est commandée, c'est-à-dire pilotée, par des moyens de traitement de signal et de pilotage 5, notamment une carte électronique à microprocesseur 6 ou analogue. Les moyens de traitement de signal et de pilotage 5 pilotent la source 1 de gaz respiratoire de manière à ce qu'elle délivre le gaz selon un (ou plusieurs) mode de ventilation prédéfinis.

De préférence, les moyens de traitement de signal et de pilotage 5 permettent de piloter la source de gaz 1 pour délivrer le gaz selon un mode ventilatoire « normal » correspondant à une ventilation d'un patient qui n'est pas en arrêt cardiaque et un mode ventilatoire « RCP » correspondant à une ventilation d'un patient qui est en arrêt cardiaque et sur lequel un secouriste entame ou pratique une RCP.

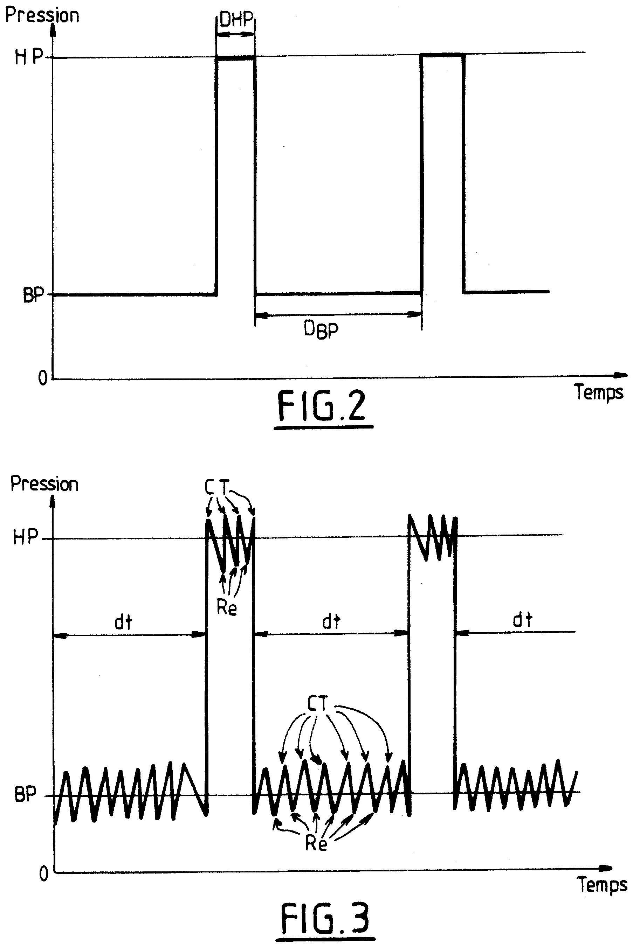

Par exemple, selon un mode de ventilation dédié à la RCP, la source 1 de gaz respiratoire est pilotée pour fournir le gaz respiratoire, typiquement de l'air, selon un cycle ventilatoire comportant plusieurs niveaux de pression ou de type « BiPAP », comme illustré en Figure 2, en particulier 2 niveaux de pression comprenant un niveau de pression basse, par exemple une pression basse (BP) comprise entre environ 0 cm H₂O et 15 cm H₂O, et un niveau de pression haute, par exemple une pression haute (HP) comprise entre environ 7 cm H₂O et 40 cm H₂O.

Le gaz est délivré alternativement entre ces deux niveaux de pression (BP, HP), comme illustré sur la Figure 2, pendant toute la réalisation de la RCP par le secouriste, c'est-à-dire pendant que le secouriste opère les CT et relâchements. La durée (D_{BP}) de fourniture du gaz à pression basse (BP) par la micro-soufflante 1 est comprise entre 2 et 10 secondes, typiquement de l'ordre de 3 à 6 secondes, alors que la durée (D_{HP}) de fourniture du gaz à pression haute (HP) est inférieure à 3 secondes, par exemple de l'ordre de 0,5 à 1,5 secondes.

La micro-soufflante 1 du ventilateur génère deux niveaux de pression, à savoir un niveau de pression haute (i.e PH) et un niveau de pression basse (i.e PB). Le massage cardiaque alternant phase de compressions thoraciques (CTs) et relâchements (Re) génère des pics de pression qui viennent se superposer aux cycles de pression du ventilateur. Il en résulte au niveau de l'interface patient, une courbe de pression comme illustré en Figure 3 où les pics de pression au niveau des plateaux hauts (i.e. à PH) et bas (i.e. à BP) reflètent les compressions thoraciques (CT) avec augmentation de pression puisque le thorax s'affaisse sous la pression de la CT opérée par le secouriste, et les relâchements (Re) avec baisse de pression puisque le thorax remonte en l'absence de CT.

Comme on le voit sur les Figures 2 et 3, dans le cadre de la présente invention, la période de temps (dt) donnée, pendant laquelle la pluralité de valeurs de teneur en CO₂ est mesurée et la valeur maximale de teneur (Vmax) en CO₂ est extraite correspond à la durée (D_{BP}) de fourniture du gaz à pression basse (BP), soit entre 2 et 10 secondes, typiquement entre 3 et 6 secondes.

Le gaz fourni par la micro-soufflante 1 est acheminé par le conduit de gaz 2 qui forme tout ou partie de la branche inspiratoire 2a du circuit patient 2a, 2b. Le gaz respiratoire, en général de l'air, est fourni au patient via une interface de distribution de gaz 3, à savoir ici une sonde d'intubation endotrachéale, plus simplement appelée 'sonde trachéale'. Toutefois, d'autres interfaces sont utilisables, notamment un masque facial ou un masque laryngé.

Le conduit de gaz 2 est en communication fluidique avec l'interface de distribution de gaz 3, telle une sonde trachéale, de manière à lui fournir le gaz provenant de la source 1 de gaz respiratoire, à savoir ici une micro-soufflante. Le conduit de gaz 2 vient en fait se raccorder à la sonde trachéale 3 par l'intermédiaire d'une pièce de raccordement intermédiaire 8, à savoir ici une pièce en Y. Cette pièce de raccordement intermédiaire 8 en Y comprend des passages de gaz internes.

La pièce de raccordement intermédiaire 8, c'est-à-dire ici la pièce en Y, est également raccordée à la branche expiratoire 2b du circuit patient 2a, 2b de manière à pouvoir recueillir et convoyer les gaz riches en CO₂ expirés par le patient P et les évacuer vers l'atmosphère (en 9).

Selon l'invention, il est également prévu des moyens de mesure de teneur en CO₂ 4, appelés capteur de CO₂ ou capnomètre, conçus pour opérer des mesures de concentration en CO₂ dans le gaz expiré par le patient P, et fournir des signaux de mesure de teneur en CO₂ aux moyens de traitement de signal et de pilotage 5 où ces signaux de mesure peuvent être traités, notamment par un (ou des) algorithme de calcul ou analogue.

Dans le mode de réalisation de la Figure 6, le capteur de CO₂ est agencé près de la bouche du patient P en configuration « flux principal » (*mainstream*), c'est-à-dire en amont et à proximité immédiate de l'interface respiratoire 3, préférentiellement entre la pièce de raccordement intermédiaire 8, i.e. la pièce en Y, et l'interface respiratoire 3, i.e. la sonde trachéale, par exemple sur une pièce de jonction 18 (cf. Fig. 6).

Selon un autre mode de réalisation (non montré), le capteur de CO₂ peut être agencé en configuration « flux dérivé » (*sidestream*). Dans ce cas, le capteur de CO₂ 4 se situe dans la carcasse de l'appareil d'assistance respiratoire et est relié, via une ligne de prélèvement de gaz, telle une tubulure ou analogue, à un site de prélèvement de gaz situé en amont et à proximité immédiate de l'interface respiratoire 3, par exemple sur la pièce de jonction 18. Cette ligne de prélèvement de gaz communique fluidiquement avec le lumen de la pièce de jonction 18 de sorte de pouvoir y prélever du gaz et l'acheminer ensuite jusqu'au capteur de CO₂ situé dans la carcasse de l'appareil.

Dans tous les cas, la pièce de jonction 18 comprend un passage interne de gaz permettant au gaz de la traverser.

Préférentiellement, le capteur de CO₂ opère des mesures en continu de la concentration en CO₂ dans le gaz circulant au travers de la pièce de jonction 18, lequel gaz est enrichi en CO₂ lors de son passage dans les poumons du patient P où s'effectue des échanges gazeux.

Les signaux de mesure de teneur en CO₂ sont ensuite transmis par le capteur de CO₂, par liaison électrique ou analogues, notamment filaire ou autre, aux moyens de traitement de signal et de pilotage 5.

En effet, le monitorage de la teneur en CO₂, en particulier de l'etCO₂ qui reflète indirectement le CO₂ alvéolaire, est d'une grande importance pendant la RCP, notamment pour détecter un RACS. En effet, un retour d'activité cardiaque spontanée (RACS), donc une augmentation significative du débit cardiaque, engendre une augmentation rapide de la quantité de CO₂ amenée par le sang aux poumons et transférée à travers la membrane alvéolo-capillaire, ce CO₂ se retrouvant ensuite dans le flux gazeux expiré par le patient.

Les moyens de traitement de signal et de pilotage 5 sont configurés (en particulier le microprocesseur 6) pour :
a) traiter les signaux de mesure de teneur en CO₂ qui correspondent à des mesures opérées par les moyens de mesure de teneur en CO₂ 4, typiquement le capnomètre ou capteur de CO₂, pendant la période de temps donnée (dt), par exemple quelques secondes, pour en extraire une pluralité de valeurs de teneur en CO₂,
b) sélectionner la valeur maximale (Vmax) de teneur en CO₂ parmi ladite pluralité de valeurs de teneur en CO₂ mesurées pendant ladite période de temps donnée (dt), et
c) transmettre cette valeur maximale (Vmax) de teneur en CO₂ à une interface graphique utilisateur 7 ou IGU.

Une source de courant électrique 10, telle une batterie rechargeable ou analogue, alimente, directement ou indirectement, en courant électrique les moyens de traitement de signal et de pilotage 5, la micro-soufflante 1, l'IGU 7 et tout autre élément de l'appareil, notamment une mémoire 11. La source de courant électrique 10 est préférentiellement agencée dans la carcasse du ventilateur.

D'une façon générale, le ventilateur médical de l'invention permet une mesure en continu de la concentration en CO₂ produit par le patient P, la mesure étant opérée par le capnomètre 4 qui est agencé sur le trajet du gaz, proche de la bouche du patient P, préférentiellement ici entre la pièce 8 en forme de Y et la sonde trachéale 3 de la Figure 6, c'est-à-dire au niveau de la pièce de jonction 18 raccordée fluidiquement entre la pièce en Y 8 et la sonde 3.

Le ventilateur permet en outre, si désiré, d'opérer en parallèle une mesure en continu des débits des gaz expirés et inspirés, à l'aide d'un ou plusieurs capteurs de débit (non montré).

Selon l'invention, l'IGU est configurée quant à elle pour afficher la valeur maximale (Vmax) de teneur en CO₂ fournie par les moyens de traitement de signal et de pilotage 5 qui est sélectionnée parmi plusieurs valeurs de concentrations en CO₂ mesurées pendant une durée donnée correspondant à plusieurs contractions thoraciques et relâchements de compression successifs opérés par un secouriste réalisant un massage cardiaque (i.e. une RCP) sur le patient P en état d'arrêt cardiaque.

En effet, la valeur de concentration en CO₂ qui reflète le plus la teneur en CO₂ alvéolaire, et donne dès lors une bonne indication de l'état de la circulation sanguine chez le patient P durant la RCP, est la valeur la plus haute de CO₂, encore appelée valeur maximale (Vmax) ou valeur de pic, comme illustré en Figure 5 qui schématise l'évolution de la teneur en CO₂ dans le gaz et illustre plusieurs mesures de l'etCO₂ mesurées pendant plusieurs durées dt successives, par exemple des durées de 3 à 6 secondes, pendant la réalisation d'une RCP. On y voit que la teneur en CO₂ du gaz n'est pas constante pendant un intervalle de temps dt donné et qu'il existe donc forcément une valeur maximale (Vmax) de teneur en CO₂ sur chaque intervalle dt, c'est-à-dire la valeur de pic.

De là, dans le cadre de la présente invention, le ventilateur mémorise (en 11) donc toutes les valeurs de pics de CO₂ pendant chaque période de temps dt, typiquement entre 3 et 7 secondes, et détermine la valeur maximale Vmax de teneur en CO₂ parmi la pluralité de pics (EtCO2_₁, EtCO2_₂, EtCO2_₃, ..., EtCO2_x) mesurés sur une période de temps (dt) donnée, comme illustré en Figure 5.

Durant une RCP, la teneur en CO₂ dans le gaz produit par le patient, et passant devant la prise de mesure du capnomètre 4 varie en fonction de la présence ou non de compressions thoraciques (CT).

Ainsi, après une insufflation d'air par la micro-soufflante 1 du ventilateur et tant que les CT n'ont pas commencé, on ne détecte pas de CO₂ dans le flux gazeux acheminé par le conduit 2 jusqu'à l'interface respiratoire 3 qui distribue ensuite cet air aux poumons du patient P.

Après quelques compressions thoraciques (CT) pratiquées par un secouriste, du CO₂ est détectée au niveau de la pièce 8 en Y par le capnomètre 4 puisque les alternances de CT et de relâchements (Re) engendrent des mouvements d'air entrant et sortant des poumons du patient P en « mimant » les phases expiratoires du patient P. De l'air expiré riche en CO₂ se retrouve alors au niveau de la pièce 8 en Y et le capnomètre 4 (cf. Fig. 6) et des mesures de concentrations en CO₂ peuvent être réalisées par le capnomètre 4. Les signaux de mesure correspondant sont envoyés aux moyens de traitement de signal et de pilotage 5 où ils sont traités comme expliqués ci-avant, de sorte à déterminer la valeur maximale (Vmax) de CO₂ sur chaque intervalle de temps dt.

La valeur maximale de CO₂ (Vmax) est celle qui représente le mieux le CO₂ alvéolaire. En effet, le CO₂ présent au niveau de la pièce 8 en Y et du capnomètre 4 est "lavé" petit à petit du fait des compressions thoraciques successives et répétées, et tend à diminuer après avoir atteint cette valeur maximale puisque les CT engendrent aussi l'évacuation vers l'atmosphère (en 9) des gaz riches en CO₂, via la branche expiratoire 2b du circuit patient. Les CT successives génèrent donc différents niveaux de CO₂, la plus représentative étant la valeur de pic ou maximale (Vmax), comme illustré en Figure 5.

Dans le cadre de la présente invention, le ventilateur mémorise (en 11) donc toutes les valeurs maximales (Vmax) de CO₂ entre deux cycles ventilatoires, c'est-à-dire pendant les durées dt successives, détermine la valeur maximale (Vmax) de teneur en CO₂ parmi la pluralité de valeurs maximales mesurées, et affiche cette valeur maximale (Vmax) à l'écran de l'IGU 7.

Cette valeur maximale (Vmax), pendant un intervalle de temps dt donné, peut être affichée en tant que valeur numérique unique. On peut aussi afficher plusieurs valeurs maximales (Vmax) mesurées successivement sur plusieurs intervalles de temps (dt) successifs. Par ailleurs, si utile ou désiré, il est aussi possible de l'afficher sous forme d'une représentation graphique présentant plusieurs valeurs maximales (Vmax) mesurées successivement sur plusieurs intervalles de temps (dt) successifs au fil du temps, par exemple sur les 2 à 5 dernières minutes, par exemple une représentation graphique de type courbe, barre-graphe, ou autre.

Les données calculées à partir de ces mesures de CO₂ permettent au secouriste de mieux « piloter » la RCP grâce à un indicateur qui reflète l'état de la circulation et du métabolisme du patient puisque, à un niveau de ventilation constant, plus la RCP est efficace plus la quantité de CO₂ produite et transférée à travers la membrane alvéolo-capillaire est importante et donc plus la quantité de CO₂ pouvant être détectée au niveau du capnomètre 4 est importante.

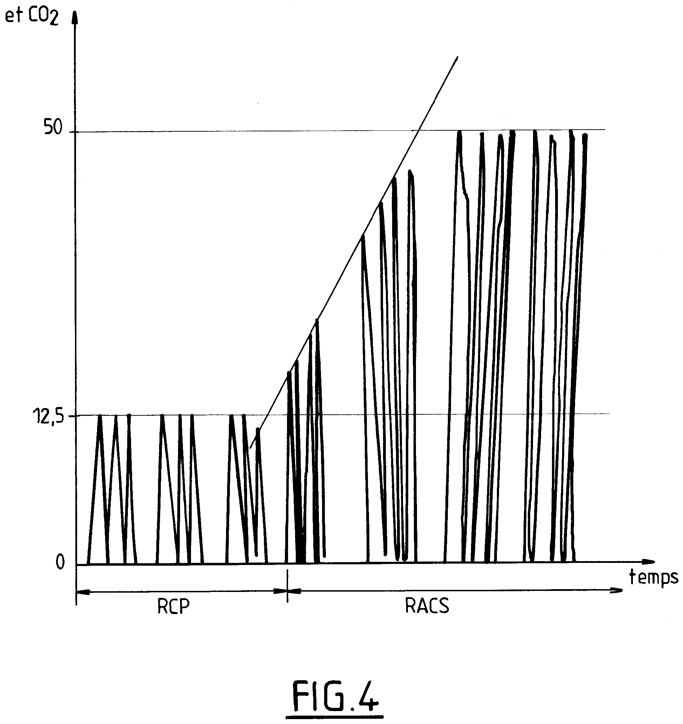

De là, en cas de retour à une activité cardiaque spontanée (RACS), la circulation reprend brutalement et donc la quantité de CO₂ alvéolaire augmente parallèlement, ce qui induit une augmentation importante la quantité de CO₂ perçu par le capnomètre 4 d'un facteur souvent supérieur à 2, comme illustré sur la Figure 4. En effet, on voit sur la Figure 4 que l'EtCO₂ est toujours inférieure à 25 KPa pendant la RCP, alors que l'EtCO₂ augmente subitement jusqu'à atteindre plus de 50 KPa lors du RACS. Ceci peut être immédiatement détecté par le secouriste qui peut alors réaliser une analyse de rythme pour stopper le massage cardiaque en cas de RACS effectif.

Dit autrement, dans le cadre de l'invention, le fait d'afficher sur l'IGU 7, la valeur maximale d'EtCO₂, pendant une période de temps donnée (dt), permet au secouriste de mieux détecter la survenance du RACS puisque cette valeur maximale (Vmax) de CO₂ reflète étroitement le CO₂ alvéolaire.

En effet, il a été constaté, dans le cadre d'essais opérés dans le cadre de la présente invention, qu'afficher en continu toutes les mesures de CO₂ ne serait pas efficace car le massage cardiaque même pratiqué de manière régulière (force de pression, fréquence...) engendre inévitablement des variations importantes de teneur en CO₂ au niveau du capnomètre, d'une CT à une autre. Cela s'expliquant par le comportement dynamique, d'ouverture/fermeture des petites voies aériennes ainsi que par l'effet de lavage de l'espace mort lors des compressions thoraciques successives entre deux cycles machine. Dès lors, afficher toutes les mesures de CO₂ pourrait induire le secouriste en erreur ou encore le « noyer » sous trop d'informations et celui-ci pourrait alors parfois « croire » à un RACS alors qu'il ne s'agirait que d'un artefact, ou à l'inverse, ne pas s'apercevoir d'un RACS chez le patient et continuer le massage alors que le patient serait en phase de RACS. Dans tous les cas, l'utilisation d'une unique valeur instantanée à des fins de pronostic ou de choix de stratégie thérapeutique est rendu risqué par le caractère oscillant de la valeur d'etCO2 instantanée, i.e. à chaque compression thoracique (CT).

Dans le cadre de l'invention, il a été montré par des essais pratiques que ces problèmes pouvaient être totalement surmontés en n'affichant que la valeur la plus élevée de teneur en CO₂ (Vmax) pendant une période de temps (dt) donnée, typiquement de quelques secondes.

En outre, il a été constaté que la teneur en CO₂ mesurée à chaque compression thoracique peut varier énormément d'une compression thoracique à l'autre. Ceci est dû non seulement à l'espace mort anatomique et instrumental mais aussi au degré d'ouverture des voies aériennes du patient. En prenant en compte ces facteurs, la valeur maximale (Vmax) de CO₂ apparaît être dès lors un meilleur reflet du CO₂ alvéolaire, et est donc un bon indicateur de RACS (si elle augmente brutalement) ou de nouvel arrêt cardiaque (si elle diminue brutalement), ce qui informe immédiatement le secouriste et de manière plus pertinente.

Ainsi, lorsque le secouriste constate une forte élévation de la valeur en CO₂ affichée, celui-ci peut en conclure que le patient est en phase de RACS, comme illustré en Fig. 4, et peut alors décider de stopper le massage cardiaque afin de procéder à une analyse de rythme par exemple.

Avantageusement, le ventilateur de l'invention peut aussi inclure des moyens d'alarme conçue et programmée pour avertir le secouriste ou analogue lorsque la valeur maximale de CO₂ mesurée excède ou, à l'inverse, devient inférieure une valeur donnée, préfixée ou calculée en continu.

En particulier, on prévoit une alarme sonore et/ou visuelle qui se déclenche lorsque la valeur maximale de CO₂ mesurée, à un instant t, est supérieure à une valeur-seuil, par exemple : [VmaxCO₂] > 1,5 x [MoyCO₂] où :
- [VmaxCO₂] est la valeur maximale de teneur en CO₂ mesurée pendant une durée dt donnée, par exemple sur une durée dt comprise entre 2 et 10 secondes,
- . [MO_{y}CO₂] est la valeur moyenne des valeurs maximales de teneur en CO₂ [VmaxCO₂] déterminées pour plusieurs durées dt successives comprises dans une fenêtre de temps (FT) donnée (FT> x.dt avec x ≥ 2), par exemple une période de 30 sec à 5 minutes, ou plus.

De façon analogue, l'alarme peut se déclencher en cas de chute brutale de la concentration en CO₂ sous une valeur minimale donnée qui pourrait être le signe d'un nouvel arrêt cardiaque du patient, d'une hyperventilation ou d'une obstruction du circuit de gaz entre le patient et la machine, par exemple un conduit flexible plié ou écrasé et ne laissant plus passer le gaz.

D'une façon générale, l'invention porte sur un ventilateur médical adapté à une utilisation pendant une réanimation cardio-pulmonaire (RCP) comprenant une source de gaz respiratoire 1, telle une micro-soufflante, des moyens de mesure de teneur en CO₂ 4, tel un capnomètre, des moyens de traitement de signal et de pilotage 5 recevant et traitant les signaux de mesure de teneur en CO₂ provenant des moyens de mesure de teneur en CO₂ 4, et une IGU 7 configurée pour afficher au moins une valeur maximale Vmax de teneur en CO₂ mesurée pendant une période de temps (dt) donnée, ladite valeur maximale Vmax de teneur en CO₂ étant sélectionnée parmi une pluralité de valeurs de teneur en CO₂ mesurées pendant ladite période de temps (dt) donnée.

L'appareil d'assistance respiratoire ou ventilateur médical selon la présente invention est particulièrement adapté à une mise en œuvre pendant une réanimation cardio-pulmonaire (RCP) sur une personne (i.e. un patient) en arrêt cardio-pulmonaire, dans le cadre de laquelle un gaz respiratoire, tel de l'air sous pression, est fourni selon un cycle ventilatoire à plusieurs niveaux de pression à ladite personne subissant le massage cardiaque avec alternance de compressions thoraciques et de relâchements. Pour faciliter son transport par les secouristes, par exemple un médecin, un infirmier, un pompier ou analogue, le ventilateur de l'invention est préférentiellement agencé dans un sac de transport.

## Revendications

1. Appareil d'assistance respiratoire pour fournir un gaz respiratoire à un patient pendant une réanimation cardio-pulmonaire (RCP) comprenant :
- une source (1) de gaz respiratoire pour fournir un gaz respiratoire à audit patient pendant une réanimation cardio-pulmonaire (RCP),
- des moyens de mesure de teneur en CO₂ (4) pour opérer des mesures de concentration en CO₂ produit par ledit patient, et fournir des signaux de mesure de teneur en CO₂ à des moyens de traitement de signal et de pilotage (5),
- des moyens de traitement de signal et de pilotage (5) configurés pour traiter les signaux de mesure de teneur en CO₂ provenant des moyens de mesure de teneur en CO₂ (4),
- au moins une interface graphique utilisateur (7),
**caractérisé en ce que** :
- les moyens de traitement de signal et de pilotage (5) sont configurés pour :
a) traiter les signaux de mesure de teneur en CO₂ correspondant à des mesures opérées par les moyens de mesure de teneur en CO₂ (4) pendant une période de temps (dt) donnée qui correspond à plusieurs contractions thoraciques et relâchements de compression successifs opérés par un secouriste réalisant la RCP sur le patient P en état d'arrêt cardiaque, et en extraire une pluralité de valeurs de pics de teneur en CO₂,
b) sélectionner la valeur maximale (Vmax) de teneur en CO₂ parmi la pluralité de valeurs de pics de teneur en CO₂ mesurées pendant ladite période de temps (dt) donnée, et
c) transmettre ladite valeur maximale (Vmax) de teneur en CO₂ à l'interface graphique utilisateur (7),
- et l'interface graphique utilisateur (7) est configurée pour afficher la valeur maximale (Vmax) de teneur en CO₂.

2. Appareil selon la revendication précédente, **caractérisé en ce que** les moyens de traitement de signal et de pilotage (5) comprennent au moins un microprocesseur.

3. Appareil selon l'une des revendications précédentes, **caractérisé en ce que** les moyens de mesure de teneur en CO₂ (4) comprennent un capnomètre.

4. Appareil selon l'une des revendications précédentes, **caractérisé en ce que** la source de gaz respiratoire (1) est en communication fluidique avec un conduit de gaz (2), ledit conduit de gaz (2) étant en communication fluidique avec une interface respiratoire (3), de préférence une sonde d'intubation endotrachéale, un masque facial ou un masque laryngé.

5. Appareil selon la revendication 4, **caractérisé en ce que** les moyens de mesure de teneur en CO₂ (4) sont agencés :
- soit en amont et à proximité immédiate (18) de l'interface respiratoire (3),
- soit dans l'appareil en étant reliés à un site de prélèvement de gaz (18) situé en amont et à proximité immédiate de l'interface respiratoire (3).

6. Appareil selon l'une des revendications précédentes, **caractérisé en ce que** la période de temps donnée (dt) est comprise entre 2 et 10 secondes, typiquement de l'ordre de 3 à 6 secondes.

7. Appareil selon l'une des revendications précédentes, **caractérisé en ce que** les moyens de mesure de teneur en CO₂ (4) sont configurés pour opérer des mesures en continu.

8. Appareil selon l'une des revendications précédentes, **caractérisé en ce qu'**il comprend des moyens de mémorisation (11) coopérant avec les moyens de traitement de signal et de pilotage (5) pour mémoriser la pluralité de valeurs de teneur en CO₂ mesurées pendant la période de temps donnée.

9. Appareil selon l'une des revendications précédentes, **caractérisé en ce qu'**il comprend des moyens d'alarme configurés pour se déclencher lorsque la valeur maximale de teneur en CO₂ excède une valeur-seuil, de préférence les moyens d'alarme comprennent une alarme sonore ou visuelle.

10. Appareil selon l'une des revendications précédentes, **caractérisé en ce que** l'interface graphique utilisateur (IGU) comprend un écran digital, de préférence un écran tactile.

11. Appareil selon l'une des revendications précédentes, **caractérisé en ce que** les moyens de traitement de signal et de pilotage (5) sont configurés pour commander la source (1) de gaz respiratoire et délivrer le gaz respiratoire selon des cycles ventilatoires successifs.

12. Appareil selon l'une des revendications précédentes, **caractérisé en ce que** la source (1) de gaz respiratoire comprend une micro-soufflante motorisée.

13. Appareil selon l'une des revendications précédentes, **caractérisé en ce que** l'interface graphique utilisateur (7) est configurée pour afficher la valeur maximale (Vmax) de teneur en CO₂ sous forme d'une valeur numérique et/ou d'une représentation graphique.

14. Appareil selon l'une des revendications précédentes, **caractérisé en ce que** les moyens de mesure de teneur en CO₂ (4) sont configurés pour opérer des mesures de concentration en CO₂ successives sur des périodes de temps (dt) successives.

## Patentansprüche

1. Beatmungseinrichtung zum Bereitstellen eines Atemgases für einen Patienten bei einer Herz-Lungen-Wiederbelebung (RCP), umfassend:
- eine Quelle (1) für Atemgas zum Bereitstellen eines Atemgases für den Patienten bei einer Herz-Lungen-Wiederbelebung (RCP),
- Mittel zum Messen des CO₂-Gehalts (4) zum Vornehmen von Messungen einer vom Patienten erzeugten CO₂-Konzentration und Bereitstellen von Messsignalen des CO₂-Gehalts für Signalverarbeitungs- und Steuerungsmittel (5),
- Signalverarbeitungs- und Steuerungsmittel (5), die konfiguriert sind, um die Messsignale des COM₂-Gehalts, die aus den Mitteln zum Messen des CO₂-Gehalts (4) stammen, zu verarbeiten,
- mindestens eine graphische Benutzeroberfläche (7),
**dadurch gekennzeichnet, dass**:
- die Signalverarbeitungs- und Steuerungsmittel (5) konfiguriert sind zum:
a) Verarbeiten der Messsignale des CO₂-Gehalts entsprechend den durch die Mittel zum Messen des CO₂-Gehalts (4) innerhalb einer gegebenen Zeitdauer (dt) vorgenommenen Messungen, die mehreren aufeinanderfolgenden thorakalen Kontraktionen und Kontraktionslockerungen entspricht, die von einem Nothelfer, der die RCP durchführt, an dem Patienten P bei Herzstillstand vorgenommen werden, und daraus Extrahieren einer Vielzahl von Werten von Spitzen eines COM₂-Gehalts,
b) Auswählen des maximalen Werts (Vmax) eines CO₂-Gehalts aus der Vielzahl von Werten von Spitzen eines COM₂-Gehalts, die innerhalb der gegebenen Zeitdauer (dt) gemessen werden, und
c) Übertragen des maximalen Werts (Vmax) eines CO₂-Gehalts zur graphischen Benutzeroberfläche (7),
- und die graphische Benutzeroberfläche (7) konfiguriert ist, um den maximalen Wert (Vmax) eines CO₂-Gehalts anzuzeigen.

2. Einrichtung nach dem vorstehenden Anspruch, **dadurch gekennzeichnet, dass** die Signalverarbeitungs- und Steuerungsmittel (5) mindestens einen Mikroprozessor umfassen.

3. Einrichtung nach einem der vorstehenden Ansprüche, **dadurch gekennzeichnet, dass** die Mittel zum Messen des CO₂-Gehalts (4) ein Kapnometer umfassen.

4. Einrichtung nach einem der vorstehenden Ansprüche, **dadurch gekennzeichnet, dass** die Atemgasquelle (1) in Fluidverbindung mit einer Gasleitung (2) ist, wobei die Gasleitung (2) in Fluidverbindung mit einer Beatmungsschnittstelle (3), vorzugsweise einer endotrachealen Tubus-Sonde, einer Gesichtsmaske oder einer Larynxmaske ist.

5. Einrichtung nach Anspruch 4, **dadurch gekennzeichnet, dass** die Mittel zum Messen des CO₂-Gehalts (4) wie folgt angeordnet sind:
- entweder stromaufwärts und in unmittelbarer Nähe (18) der Beatmungsschnittstelle (3),
- oder in der Einrichtung, indem sie an eine Gasentnahmestelle (18) angeschlossen sind, die sich stromaufwärts und in unmittelbarer Nähe der Beatmungsschnittstelle (3) befindet.

6. Einrichtung nach einem der vorstehenden Ansprüche, **dadurch gekennzeichnet, dass** die gegebene Zeitdauer (dt) zwischen 2 und 10 Sekunden, typischerweise in der Größenordnung von 3 bis 6 Sekunden, enthalten ist.

7. Einrichtung nach einem der vorstehenden Ansprüche, **dadurch gekennzeichnet, dass** die Mittel zum Messen des CO₂-Gehalts (4) konfiguriert sind, um kontinuierlich Messungen vorzunehmen.

8. Einrichtung nach einem der vorstehenden Ansprüche, **dadurch gekennzeichnet, dass** sie Speichermittel (11) umfasst, die mit den Signalverarbeitungs- und Steuerungsmitteln (5) zusammenwirken, um die Vielzahl von Werten eines COM₂-Gehalts, die innerhalb der gegebenen Zeitdauer gemessen werden, zu speichern.

9. Einrichtung nach einem der vorstehenden Ansprüche, **dadurch gekennzeichnet, dass** sie Alarmmittel umfasst, die konfiguriert sind, um auszulösen, wenn der maximale Wert eines CO₂-Gehalts einen Schwellenwert überschreitet, wobei die Alarmmittel vorzugsweise einen akustischen oder optischen Alarm umfassen.

10. Einrichtung nach einem der vorstehenden Ansprüche, **dadurch gekennzeichnet, dass** die graphische Benutzeroberfläche (IGU) einen digitalen Bildschirm, vorzugsweise einen berührungsempfindlichen Bildschirm, umfasst.

11. Einrichtung nach einem der vorstehenden Ansprüche, **dadurch gekennzeichnet, dass** die Signalverarbeitungs- und Steuerungsmitteln (5) konfiguriert sind, um die Quelle (1) für Atemgas zu steuern und das Atemgas gemäß aufeinanderfolgenden Beatmungszyklen abzugeben.

12. Einrichtung nach einem der vorstehenden Ansprüche, **dadurch gekennzeichnet, dass** die Quelle (1) für Atemgas ein motorisiertes Mikrogebläse umfasst.

13. Einrichtung nach einem der vorstehenden Ansprüche, **dadurch gekennzeichnet, dass** die graphische Benutzeroberfläche (7) konfiguriert ist, um den maximalen Wert (Vmax) eines CO₂-Gehalts in Form eines Zahlenwerts und/ oder einer graphischen Darstellung anzuzeigen.

14. Einrichtung nach einem der vorstehenden Ansprüche, **dadurch gekennzeichnet, dass** die Mittel zum Messen des CO₂-Gehalts (4) konfiguriert sind, um aufeinanderfolgende Messungen einer CO₂-Konzentration über aufeinanderfolgende Zeitdauern (dt) vorzunehmen.

## Claims

1. Respiratory assistance apparatus for providing respiratory gas to a patient during cardiopulmonary resuscitation (CPR) comprising:
- a source (1) of respiratory gas for providing respiratory gas to said patient during cardiopulmonary resuscitation (CPR),
- CO₂ content measuring means (4) for carrying out concentration measurements of the CO₂ produced by said patient, and providing CO₂ content measurement signals to signal processing and control means (5),
- signal processing and control means (5) configured to process the CO₂ content measurement signals provided by the CO₂ content measuring means (4),
- at least one graphical user interface (7),
**characterised in that**:
- the signal processing and control means (5) are configured to:
a) process the CO₂ content measurement signals corresponding to measurements carried out by the CO₂ content measuring means (4) during a given time period (dt) which corresponds to several successive thoracic contractions and compression releases performed by a first-aider carrying out CPR on the patient P in a state of cardiac arrest, and to extract therefrom a plurality of values of CO₂ content peaks,
b) select the maximum value (Vmax) of CO₂ content among the plurality of values of CO₂ content peaks measured during said given time period (dt), and
c) transmit said maximum value (Vmax) of CO₂ content to the graphical user interface (7),
- and the graphical user interface (7) is configured to display the maximum value (Vmax) of CO₂ content.

2. Apparatus according to the preceding claim, **characterised in that** the signal processing and control means (5) comprise at least one microprocessor.

3. Apparatus according to one of the preceding claims, **characterised in that** the CO₂ content measuring means (4) comprise a capnometer.

4. Apparatus according to one of the preceding claims, **characterised in that** the source of respiratory gas (1) is in fluidic communication with a gas conduit (2), said gas conduit (2) being in fluidic communication with a respiratory interface (3), preferably an endotracheal intubation tube, a face mask or a laryngeal mask.

5. Apparatus according to claim 4, **characterised in that** the CO₂ content measuring means (4) are arranged:
- either upstream and in immediate vicinity (18) of the respiratory interface (3),
- or in the apparatus and being linked to a gas sampling site (18) located upstream and in immediate vicinity of the respiratory interface (3).

6. Apparatus according to one of the preceding claims, **characterised in that** the given time period (dt) is comprised between 2 and 10 seconds, typically around 3 to 6 seconds.

7. Apparatus according to one of the preceding claims, **characterised in that** the CO₂ content measuring means (4) are configured to carry out measurements continuously.

8. Apparatus according to one of the preceding claims, **characterised in that** it comprises storage means (11) engaging with the signal processing and control means (5) to store the plurality of values of CO₂ content measured during the given time period.

9. Apparatus according to one of the preceding claims, **characterised in that** it comprises alarm means configured to be triggered when the maximum value of CO₂ content exceeds a threshold value, preferably the alarm means comprise an audible or visual alarm.

10. Apparatus according to one of the preceding claims, **characterised in that** the graphical user interface (GUI) comprises a digital screen, preferably a touchscreen.

11. Apparatus according to one of the preceding claims, **characterised in that** the signal processing and control means (5) are configured to control the source (1) of respiratory gas and deliver the respiratory gas according to successive ventilatory cycles.

12. Apparatus according to one of the preceding claims, **characterised in that** the source (1) of respiratory gas comprises a motorised micro-blower.

13. Apparatus according to one of the preceding claims, **characterised in that** the graphical user interface (7) is configured to display the maximum value (Vmax) of CO₂ content in the form of a numerical value and/or a graphical representation.

14. Apparatus according to one of the preceding claims, **characterised in that** the CO₂ content measuring means (4) are configured to carry out successive CO₂ concentration measurements in successive time periods (dt).
